# EUROPEAN PATENT SPECIFICATION

(11) **EP 1 696 873 B1**
(45) Date of publication and mention of the grant of the patent: **21.06.2017**
(21) Application number: 03800190.5
(22) Date of filing: 23.12.2003
(51) Int. Cl.: A61K 9/00, C07C 67/54, C07C 67/03, C07C 67/333, C07C 69/587, C11B 3/12, C11C 3/10, C11C 3/14, C11C 1/02, C11C 1/10, C11C 3/00

(54) **PRODUCTION AND PURIFICATION OF ESTERS OF CONJUGATED LINOLEIC ACIDS**
HERSTELLUNG UND REINIGUNG VON ESTERN AUS KONJUGIERTEN LINOLSÄUREN
FABRICATION ET PURIFICATION D'ESTERS D'ACIDES LINOLEIQUES CONJUGUES

(43) Date of publication of application: 06.09.2006
(73) Proprietor: STEPAN COMPANY, Northfield, Illinois 60093 (US)
(72) Inventor: RONGIONE, Joseph, C., Highlands, NJ 07732 (US); GALANTE, Jenifer, Heydinger, Oakland, NJ 07436 (US); CLAUSS, Steven, L., Manville, NJ 08835 (US); BERNHARDT, Randal, J., Antioch, IL 60002 (US); XAYARIBOUN, Phouvieng, North Aurora, IL 60542 (US)
(74) Representative: Schnappauf, Georg
(86) International application number: PCT/US2003/041289
(87) International publication number: WO 2005/067888

(56) References cited:
- WO-A-00/09163
- WO-A-03/043972
- WO-A2-01/40419
- US-A- 3 674 731
- US-A1- 2003 181 522
- US-B1- 6 479 683

## Description

### FIELD OF THE INVENTION

The invention relates to improved methods for the manufacture of conjugated linoleic acid-containing materials which decrease the formation of undesirable conjugated linoleic acid isomers; decrease the formation of unconjugated fatty acid esters; reduce or remove unwanted ester side products and components; decrease processing time; decrease process stream color; improve oxidative stability of conjugated linoleic acid esters; streamline the production of conjugated linoleic acid esters; and/or decrease process waste streams.

### BACKGROUND OF THE INVENTION

Conjugated linoleic acids (CLAs) refer to a mixture of positional and geometric isomers of linoleic acids, i.e., octadecadienoic acids, which are unsaturated fatty acids considered essential to the human diet and found preferentially in dairy products and meat. CLAs have generated much interest in the academic and business communities because of their nutritional, therapeutic, and pharmacological properties. There are numerous known CLA compositions, along with various known methods to prepare and/or purify such compositions. *See, e.g.,* U.S. Pat. Nos. 6,420,577 (Reaney, et al.); 6,015,833 (Saebo, et. al.); 6,160,140 (Bhaggan, et. al.); 6,034,132 and 6,019,990 (both to Remmereit, J.); 6,225,486 (Saebo, et. al.), and WO 02/022768 (Cognis Deutschland GmbH & Co.). CLAs have become biologically and commercially important, as they have been observed to inhibit mutagenesis and to provide unique nutritional value.

Typically, CLAs are a mixture of positional isomers of linoleic acids (C18:2) having conjugated double bonds. The cis-9, trans-11 (c9,t11) and trans-10, cis-12 (t10,c12) isomers are present in greatest abundance in typical CLA compositions, but it is still uncertain to those in the art which isomers are responsible for the biological and heightened nutritional activity observed with such mixtures. However, it has been noted from previous labeled uptake studies that the 9,11-isomer appears to be somewhat preferentially taken up and incorporated into the phospholipid fraction of animal tissues; and to a lesser extent the 10,12-isomer has been found to be similarly incorporated. *See* Ha, et al., Cancer Res., 50:1097 (1991). Others have reported that virtually all of the biological activity of the mixed CLA isomers could be attributed to the t10,c12-CLA isomer while very little activity could be ascribed to the c9,t11-CLA isomer. *See* Sebedio et al., Inform Vol. 10, No. 5.

The properties of unsaturated fatty acids and their derivatives can be altered by rearrangement, i.e., isomerization of the structure of the double bonds, either with respect to their steric positions or the positions of the double bonds in the carbon chain of a fatty acid molecule. As noted above, conjugated fatty acid derivatives are of great technical and commercial interest and, therefore, many attempts have been made to isomerize unconjugated fatty acids into conjugated fatty acids. Without being bound by any particular theory, it is believed that the a shifting of the double bonds within a linoleic acid is possible because the conjugated form of the linoleic acid has a lower state of energy than the unconjugated form.

Previously known methods to produce conjugated unsaturated compounds include, for example, hydrogenation of fats using a variety of catalysts. Such a method, however, often lead to incomplete isomerization and unwanted side reactions, such as polymerization and intramolecular cyclization. Other known methods, for example, include isomerization with an excess of alkali metal hydroxide in an aqueous or alcoholic medium, which leads to a quantitative isomerization. However, this particular method typically suffers from the limitation that a considerable excess of alkali metal hydroxide must be utilized so that the conjugated fatty acids or fatty acid compounds are obtained in the form of alkali soaps. Moreover, the resultant conjugated fatty acids or fatty acid compounds have to be recovered and isolated from the mixture. These techniques also differ in their use of a particular solvent, temperature and pressure. *See, e.g.,* U.S. Pat. No. 3,162,658 (Baltes, et. al.).

It has also been shown that the rearrangement of the double bonds of linoleic acids to conjugated positions can occur during treatment with catalysts such as nickel or alkali at high temperatures, and during autooxidation. It is theoretically possible that eight geometric isomers of 9,11 and 10,12 octadecadienoic acid (c9,c11; c9,t11; t9,c11; t9,t11; c10,c12; c10,t12; t10,c12 and t10,t12) could result from the isomerization of c9,c12-octadecadienoic acid. Again, without being bound by any particular theory, a general mechanism for the isomerization of linoleic acids has been described by J. C. Cowan in JAOCS 72:492-99 (1950). The formation of certain isomers of CLAs is thermodynamically favored as described therein. The relatively higher distribution of 9,11 and 10,12 isomers apparently results from the further stabilization of the c9,t11 or t10,c12 geometric isomers.

U.S. Pat. No. 6,160,140 (Bhaggan, et al., the '140 patent) discloses the conversion of a linoleic acid-containing oil, free fatty acid or alkyl ester to CLAs by treating such compositions with a base in an alcohol solution, where the alcohol has at least 3 carbons and at least 2 hydroxyl groups. Preferably, the '140 patent utilizes potassium hydroxide in propylene glycol. The use of a solvent in the conjugation (isomerization) step gives rise to the potential formation of unwanted CLA-alcohol esters (e.g. CLA-propylene glycol esters).

U.S. Pat. No. 3,984,444 (Ritz, et al., the '444 patent) describes the isomerization of an ester of an alcohol having 1 to 12 carbon atoms and a fatty acid having 10 to 24 carbon atoms with isolated double bonds, to the corresponding compound having conjugated double bonds, through the use of an alkaline metal alcoholate in a strongly polar aprotic solvent. As noted above, the use of a solvent in the conjugation step is undesirable.

As previously described, CLAs have a wide variety of nutritional, therapeutic, and pharmacological uses. Those uses include, for example, body fat reduction, body weight reduction, increased muscle mass, increased feed efficiency, attenuated allergic reactions, prevention of weight loss due to immune stimulation, elevated CD-4 and/or CD-8 cellular counts in animals, increased bone mineral content, prevention of skeletal abnormalities in animals and/or decreased blood cholesterol levels.

The anticarcinogenic properties of CLAs have also been well documented. Administration of CLAs inhibits rat mammary tumorigenesis, as demonstrated by Ha, et al., Cancer Res., 52:2035s (1992). Ha, et al., Cancer Res., 50:1097 (1990) reported similar results in a mouse forestomach neoplasia model as well. CLAs have also been identified as a strong cytotoxic agent against target human melanoma, colorectal and breast cancer cells in vitro. A major review article confirms the conclusions drawn from individual studies. *See* Ip, Am. J. Clin. Nutr., 66 (6 Supp.): 1523s (1997).

More recently, much attention has focused on CLAs nutritively as a dietary supplement. CLAs have been found to exert a profound generalized effect on body composition, in particular with respect to redirecting the partitioning of fat and lean tissue mass. *See,* e.g., U.S. Pat. No. 5,554,646 (Cook, et al.), which discloses a method utilizing CLAs as a dietary supplement in various mammals, wherein a significant drop in fat content was observed with a concomitant increase in protein mass. *See also,* U.S. Pat. No. 5,428,072 (Cook, et al.) which discloses that incorporation of CLAs into animal feed (birds and mammals) increases the efficiency of feed conversion leading to greater weight gain in the CLA supplemented animals. Thus, the potential beneficial effects of CLA supplementation for food animal growers are apparent.

U.S. Pat. Nos. 6,203,843 and 6,042,869 (both to Remmereit, J.) disclose bulk animal feeds containing CLAs. U.S. Pat. Nos. 6,242,621 (Jerome et. al.) and 6,333,353 (Saebo, et. al.) both disclose isomer enriched CLA compositions and methods of preparing such compositions.

CLAs are naturally occurring in foods and feeds consumed by humans and animals alike. In particular, CLAs are abundant in products from ruminants. For example, several studies have been conducted in which CLAs have been surveyed in various dairy products. Aneja, et al., J. Dairy Sci., 43:231 (1990) observed that the processing of milk into yogurt resulted in a concentration of CLAs. Linoleic acid is an important component of biolipids, and comprises a significant proportion of triglycerides and phospholipids. Linoleic acid is also known as an "essential" fatty acid, meaning that the animal must obtain it from exogenous dietary sources because it cannot be autosynthesized.

The problem with most CLA products (which include CLAs and CLA derivatives) made by conventional approaches is their heterogeneity and the substantial variation in isoform from batch to batch. Considerable attention has been given to the fact that the ingestion of large amounts of hydrogenated oils and shortenings, instead of animal tallow, has resulted in a diet high in trans-fatty acid content. For example, Holman, et al., PNAS, 88:4830 (1991) describes rats that had been fed hydrogenated oils to give rise to an accumulation in the rats' livers of unusual polyunsaturated fatty acid isomers, which appeared to interfere with the normal metabolism of naturally occurring polyunsaturated fatty acids. These concerns were summarized in an early Editorial in Am. J. Public Health, 84:722 (1974).

Another problem with most CLA products made by conventional approaches is that they have a color, normally a straw yellow color, and contain impurities such as metal ions, malonate derivatives, etc. The yellow color detracts from marketability of the CLA products while the metal ions can cause the products to be unstable. In traditional CLA products, antioxidants are added to improve the oxidative stability of CLAs, CLA esters, or other CLA derivatives.

Therefore, there exists a need for an improved process to produce a superior CLA composition, which is enriched with highly desired c9,t11- and t10,c12-CLA isomers; which is low in certain undesirable CLA isomers and unwanted ester side products; which is clear in color; or which has increased oxidative stability. Additionally, there is a need for an improved process to readily and economically prepare such CLA compositions in a safer and more environmentally friendly way.

Prior art describes two basic processes to convert linoleic-rich oil to CLAs. In one process, the linoleic acid-rich oil undergoes a concurrent saponification/isomerization at an elevated temperature (typically 170-200 °C) in the presence of a hydroxide in a suitable solvent. Formation of unwanted CLA isomers from thermal rearrangement of the double bonds in the c9,t11- and t10,c12-isomers of CLA has been observed in this temperature range. This process also suffers in that all of the purification is performed on the acid form of CLAs, the least suitable derivative of CLA for purification. Such an unwanted result is due to the acid form's higher boiling point and higher susceptibility to oxidation, relative to methyl and ethyl ester derivatives. Additionally, other available purification methods for the acid form of CLAs are complex or inefficient.

For example, U.S. Pat. No. 6,420,577 (Reaney, et al., the '577 patent) describes a process for making CLAs via simultaneous hydrolysis and isomerization by reacting a linoleic acid-rich oil with a base in the presence of a catalytic amount of such a base that is in an aqueous medium. This process utilizes a heightened temperature (>170 °C), which leads to the formation of undesirable CLA isomers, including the trans, trans-CLA isomers. The '577 patent also discloses the use of solid phase refining methods to refine the produced CLAs, for example, using crystallization from organic solvents to partially enrich and concentrate specific CLA isomers. However, Example 19 of the '577 patent sets forth that a distillation process "was not an appropriate method of refining CLA" because large amounts of undesirable CLA by-products of unknown biological activity were formed during the distillation.

The second general process to produce CLAs involves the conversion of the linoleic acid-rich oil to an alkyl ester that is purified by some method (usually distillation), and then the ester is further isomerized. As mentioned above, there is the potential for thermal rearrangement of the double bonds of a CLA if the ester is isomerized at an elevated temperature using hydroxide. Should the isomerization of the purified linoleate ester be effected by an alkoxide, the resulting CLA ester will contain color bodies and malonate impurities.

Conventionally, it is believed that esters of CLAs undergo thermal rearrangement if purified via a distillation step due to the prolonged heating in a distillation unit because the thermal stability of unconjugated linoleate esters is believed to be superior to that of the corresponding conjugated linoleate esters. Therefore, in the prior art, distillation is done prior to isomerization.

WO 02/022768 (Cognis Deutschland GmbH & Co.) and US Pat. No. 6,225,486 (Saebo, et. al.) are examples of the prior art for the purification of alkyl esters of unconjugated linoleic acids, i.e., linoleate esters. The linoleate esters were formed by transesterification of a linoleic acid-rich oil. After purification, such linoleate esters were isomerized to give conjugated linoleic esters. However, the described isomerization process generated color bodies and malonate side products. Pre-isomerization distillation cannot reduce or remove these side products from the CLA ester product. Also, unconjugated linoleate esters (*cis-cis, cis-trans* and *trans-cis*) are left in the ester stream.

U.S. Pat. No. 3,162,658 (Baltes, et. al., the '658 patent) describes the use of alkali metal hydrocarbyl alcoholates or alkali metal amides to isomerize esters of unconjugated polyethylene acids such as linoleic acids. The '658 patent further describes the distillation of the alkyl esters of linoleic acid prior to the isomerization reaction. Furthermore, the '658 patent provides for the utilization of polar solvents for the isomerization step, which is undesirable.

WO03043972 (Saebo and al) discloses the purification of a conjugated linoleic acid stream in a molecular distillation plant.

WO0009163 discloses a process for the production and the purification of conjugated linoleic-acid containing material.

### BRIEF SUMMARY OF THE INVENTION

In one aspect, there is provided a method to refine CLA products by distilling an ester stream containing CLA esters. The CLA ester stream to be distilled can be produced by any method disclosed herein or that is currently known or will be known in the art. Distillation of the present embodiment can be done by a single or multi-pass distillation operation. The distillation apparatus used is a thin-film or wiped-film evaporator and contains a fractionating column. During the distillation operation, thermal rearrangement of CLA components is prevented or at least reduced by the use of a low residence time distillation apparatus. Furthermore, the distillation apparatus can be operated at a reduced pressure.

By utilizing a distillation operation, the peroxide value of the CLA ester stream can be significantly reduced. Additionally, the presently described distillation operation can at least partially remove the side products generated during the formation of the CLA esters. Such side products, include but are not limited to color bodies, malonate derivatives, residual glycerides, and unwanted conjugated linoleic acid isomers. Other unconjugated linoleic acid components in the CLA ester stream can also be at least partially removed via this distillation operation. The described distillation operation can also improve the oxidative stability of the distilled CLA esters and CLA derivatives produced therefrom without the addition of antioxidants.

Another aspect provides a process to produce refined CLA products. In accordance with this aspect , an alkyl ester composition containing alkyl esters of linoleic acid-containing fatty acids is first generated by transesterification of a linoleic acid-containing oil, which is then isomerized to form a CLA-containing fatty acid ester stream, i.e., an ester stream containing conjugated linoleate esters. Transesterification in this technology refers to the substitution of one alcohol moiety for another. For example, conversion of an oil to a methyl ester involves replacing the glycerol portion of the oil with methanol.

The aspect further provides that the CLA-containing fatty acid ester stream is distilled via a distillation apparatus which is a thin-film or wiped-film evaporator and contains a fractionating column to produce a refined CLA-containing fatty acid ester stream that is enriched in CLA esters.

The isomerization step can be performed at temperatures low enough to suppress formation of undesirable CLA isomers, but sufficient to cause rearrangement of the double bonds.

Moreover, the isomerization can be catalyzed by a base in a nonaqueous system. The base catalyst can be, but is not limited to, an alkyl alcoholate such as an alkali or alkaline earth alkoxide salt of a lower alkyl group alcohol having 1-4 carbons. The cation of the alkoxide salt can be sodium, potassium or calcium. The base catalyst can be delivered as a solid or as a solution in the conjugate alcohol of the alkoxide, and preferably, the catalyst is added to the alkyl ester of the linoleic acid-rich fatty acid at or below 140 °C. The preferred operating temperature for this isomerization step is in the range of 90-140 °C, more preferably in the range of 110-120 °C. Furthermore, the preferred catalyst loading is 1-4% by weight based on the weight of the linoleic acid-containing material.

Any linoleic acid-containing oil, such as safflower oil, corn oil, sunflower oil, soybean oil, cottonseed oil, sesame oil, grape seed oil, derivatives, or combinations thereof can be used in the practice of the described technology.

The transesterification and isomerization steps can be performed in one reaction vessel concurrently or sequentially without an intervening distillation step purifying the alkyl ester composition.

Additionally, the transesterification and isomerization steps can occur concurrently in a continuous reaction system using a dual reaction zone apparatus. Moreover, the transesterification side products can be removed via a centrifuge, an in-line phasing unit, or a separate phasing vessel. Further, in accordance with this embodiment, the transesterification reaction can occur in a first reaction zone, and the isomerization process can be completed in a second reaction zone.

The CLA-containing fatty acid ester stream produced from the isomerization step can be distilled at a reduced pressure, to provide a substantially clear CLA-containing fatty acid ester stream enriched in the conjugated linoleate component, which can then be further processed to produce CLAs, CLA derivatives and other CLA products.

### DETAILED DESCRIPTION OF THE INVENTION

### Definitions And Conventions

As used herein, the term "conjugated linoleic acid(s)" or "CLA(s)" refers to any conjugated linoleic acid or octadecadienoic free fatty acid. It is intended that this term encompasses all geometric isomers of linoleic acid with two conjugated carbon-carbon double bonds at any position in the respective molecule. A CLA differs from an ordinary linoleic acid in that an ordinary linoleic acid has double bonds at carbon atoms 9 and 12 while a CLA has conjugated double bonds. Examples of CLAs include, but are not limited to, cis- and trans- isomers ("E/Z isomers") of the following positional isomers: 2,4-octadecadienoic acid, 4,6-octadecadienoic acid, 6,8-octadecadienoic acid, 7,9-octadecadienoic acid, 8,10-octadecadienoic acid, 9,11-octadecadienoic acid, 10,12-octadecadienoic acid, and 11,13-octadecadienoic acid. As used herein, the term "CLA(s)" encompasses a single isomer, a selected mixture of two or more isomers, and a non-selected mixture of isomers obtained from natural sources, as well as synthetic and semisynthetic CLAs.

The term "CLA derivatives" refers to moieties of CLAs recognized by one skilled in the art as structures that can be readily converted to carboxylic acids. Examples of such moieties are carboxylic acids, salts of carboxylic acids, carboxylic anhydrides, amides, carboxylic esters, ortho esters, 1,3-dioxolanes, dioxanones, oxazoles and hydrazides.

As used herein, it is intended that the term "esters" of CLA (or "CLA esters") include any and all positional and geometric isomers of CLA bound through an ester linkage to an alcohol or any other chemical group, including, but not limited to, physiologically acceptable, naturally occurring alcohols (e.g., methanol, ethanol, propanol). Therefore, an ester of CLAs or an esterified CLA or a CLA ester may contain any of the positional and geometric isomers of CLAs.

It is intended that the term "undesirable isomers" of CLAs includes, but is not limited to, c11,t13-; t11,c13-; t11,t13-; c11,c13-; c8,t10-; t8,t10-; and c8,c10- isomers of octadecadienoic acids, but does not include t10,c12- and c9,t11-isomers of octadecadienoic acids. Undesirable isomers may also be referred to as "minor isomers" of CLAs as these isomers are generally produced in low amounts when CLAs are synthesized by alkali isomerization.

As used herein, the term "c" encompasses a chemical bond in the cis orientation, and the term "t" refers to a chemical bond in the trans orientation. If a positional isomer of CLA is designated without a "c" or a "t", then that designation includes all four possible isomers. For example, 10,12 octadecadienoic acid encompasses c10,t12-; t10,c12-; t10,t12-; and c10,c12-octadecadienoic acid, while t10,c12-octadecadienoic acid or t10,c12-CLA refers to just the single isomer.

As used herein, the term "oil" refers to a free flowing liquid containing long chain fatty acids (e.g., linoleic acids and CLAs) or other long chain hydrocarbon groups, which can comprise triglycerides of CLAs and linoleic acids. The long chain fatty acids, include, but are not limited to, the various isomers of CLAs.

Additionally, as used herein, it is intended that the term "triglycerides" of CLAs (or linoleic acids) may contain CLAs (or linoleic acids) at any or all of the three positions on the triglyceride backbone. Moreover, a triglyceride of CLA may contain any of the positional and geometric isomers of CLAs.

Furthermore, as used herein, a "linoleic acid-rich/containing" (or "CLA-rich/containing") material is a material-which can be an oil, an ester, a salt or other derivatives thereof-that is rich in or contains linoleic residues (or CLA residues). A "linoleic acid residue" (or "CLA residue") means a component which has a fatty carbon chain length and isomer distribution that resembling linoleic acids (or CLAs).

It should be understood that the fatty acid distribution in the examples of the present application was determined by gas chromatography (GC) using a Chrompack CP-Sil 88 capillary column (100 m x 0.25 mm, df = 0.2 microns) using helium carrier at approximately 1.0 mL/minute. And the following temperature parameters were used: injector at 250 °C; detector at 250 °C; oven temperature at 75 °C for 2.0 minutes (min), then increased at 5 °C/min to 185 °C and held for 30.0 min, then increased at 4 °C/min to 225°C and held for 36.0 min.

### Description Of The Invention

The presently described technology encompasses a process to use distillation to refine CLA esters. It also encompasses a process to produce CLA esters by transesterification and isomerization, and then subsequently refine the CLA esters by distillation.

The presently described technology also provides a process to distill an ester stream containing CLA esters to produce a refined ester stream enriched in esters of desirable CLA isomers. Although not wanting to be bound by any particular theory, it is believed that the data and examples supplied herein show that distillation of the conjugated linoleate esters is more efficient than the distillation of linoleate esters. Other constituent fatty acid esters found in CLA ester streams are more readily removed from the CLA esters via distillation than from the corresponding unconjugated linoleate esters.

More specifically, the distillation operation of the present technology can be a single or multi-pass distillation operation. Such distillation operations can include a batch distillation or one in which the stream to be distilled is fed to a wiped-film distillation apparatus a number of times, with each pass increasing the content of the CLA derivatives. The final pass of such a run can be used to remove color and impurities with a higher boiling point than that of the CLA derivatives.

During the distillation operation, a thin-film or wiped-film evaporator can be used to prevent or at least reduce thermal rearrangement of CLA components. Such a distillation apparatus can also be operated at a reduced pressure of lower than 101.325 kPA [760 mmHg], preferably from about 0.0013 kPA to about 6.666 kPA [about 0.01 mmHg to about 50 mmHg], more preferably from about 0.007 kPA to about 2.666 kPa [about 0.05 mmHg to about 20 mmHg], more preferably from about 0.0133 kPa to about 1.333 kPa [about 0.1 mmHg to about 10 mmHg]. An example of a suitable low residence time distillation apparatus to one of ordinary skill in the art is a hybrid wiped-film/fractional still system supplied by Pope Scientific, Inc. (Saukville, WI).

In such a system, only a small portion (typically 0.1-5%) of the total feed quantity is heated at any given moment during the run. This greatly reduces the cumulative detrimental effects of exposure of the feedstock to elevated temperatures. In contrast, a batch distillation exposes the entire feedstock, or that which remains in the vessel, to elevated temperatures for the duration of the distillation run.

In one embodiment of the present technology, the distillation operation can significantly reduce the peroxide value of the CLA ester stream. In another embodiment, undesired side products, such as residual glycerides, color bodies, malonate derivatives, unreacted unconjugated linoleic species, unwanted CLA isomers generated during the formation of the CLA ester stream (e.g., during the transesterification and isomerization steps) can be substantially removed with a single distillation. Other non-CLA components in the CLA ester stream can also be substantially removed via distillation. The distillation operation of the presently described technology can also improve the oxidative stability of the distilled CLA esters and CLA derivatives produced from the distilled CLA esters without the addition of antioxidants.

Co-pending U.S. Patent Application No. 10/434,011 provides a process to produce a CLA product enriched in desired CLA isomers, which include the following steps, isomerization of an alkyl ester of a linoleic acid-containing material (such as sunflower oil or safflower oil) to effectuate conjugation of the double bonds; saponification of the resultant CLA-containing fatty acid ester to produce a CLA-containing fatty acid salt; and then optionally neutralization of the CLA-containing fatty acid salt with an acid source to produce a CLA-containing fatty acid.

The distillation operation of the present technology can also be used in combination with the process disclosed in U.S. Patent Application No. 10/434,011 as an intervening step between the isomerization and the saponification steps. But, the CLA ester stream to be distilled can be produced or prepared in any method disclosed in this application or that is currently known or will be known in the art.

In accordance with another aspect of the presently described technology, an alkyl ester composition containing alkyl esters of linoleic acid-rich fatty acids is generated by the transesterification of a linoleic acid-rich oil. The alkyl ester composition is isomerized to form a fatty acid ester stream rich in CLA esters, i.e., conjugated linoleate esters. This CLA-rich fatty acid ester stream is then distilled via a thin film or wiped film evaporator at a reduced pressure of, for example, about 0.0133 - 1.333 kPa [about 0.01-10 mmHg]. The new ester stream resulting from the distillation operation is enriched in esters of desirable CLA isomers and contains a reduced amount of side products or impurities.

The alkyl ester composition used in the process of the invention is derived from a suitable fatty oil. Such oils include, for example, those which are naturally high in linoleic acid residues, such as safflower oil, corn oil, sunflower oil, soybean oil, grape seed oil, cottonseed oil, sesame oil, combinations of such oils, or derivatives thereof. Typically, fatty oils are triglycerides which can be wholly or substantially converted to an alkyl ester material by transesterification. Such alkylation can be accomplished by known esterification routes using short chain C₁ - C₆ alcohols or any other suitable alcohol. The resulting alkyl ester may be known as a lower alkyl ester. During the transesterification (sometimes also called alkylation or interesterification) step, triglycerides in a suitable fatty oil are converted to the alkyl ester composition containing alkyl linoleate esters. This alkyl ester composition may also contain small amounts of incompletely transesterified monoglycerides and/or diglycerides along with a significant amount of glycerine.

In addition, the isomerization step is typically catalyzed by a base in a nonaqueous system, and the catalyst can be an alkali or alkaline earth alkoxide salt of an alkyl group alcohol, i.e., alkyl alcoholates, or alkali or alkaline earth metal amides. Any alkali or alkaline earth metal compound of any monohydric alcohol can be used as a catalyst for the isomerization step of the present technology described herein. Examples of such alkyl alcoholates catalysts are alcoholates of monohydric alcohols with 1-18 carbon atoms of the alkali or alkaline earth metals. Such alkali or alkaline earth metal alcoholates include, but are not limited to, alcoholates of methyl, ethyl, propyl, butyl, tertiary butyl, lauryl, stearyl, oleyl, or benzyl alcohols. The specific alcoholates set forth in this paragraph except those derived from benzyl alcohol can be termed as alkali or alkaline earth metal alcoholates. Alkali or alkaline earth metal alcoholates can also be called alkali or alkaline earth metal hydrocarbyl alcoholates. Cesium, rubidium, potassium, sodium, calcium, lithium, magnesium or zinc alcoholates are typically utilized, along with mixtures of such alcoholates.

Substances such as alkali or alkaline earth metals, alkali or alkaline earth metal hydrides, and other organic alkali or alkaline earth metal compounds, e.g., triphenyl sodium, may also be used in accordance with the presently described technology so long as they react in the reaction mixture to form active catalysts such as alkali or alkaline earth metal alcoholates or alkali or alkaline earth metal amides.

Sodium (Na), potassium (K) or calcium (Ca) alkoxide salts of lower alkyl group alcohols (1-4 carbons) are preferred. The catalyst loading can be 1-7% by weight, alternatively 1-4% by weight, alternatively 1.8-3 % by weight, based on the weight of the alkyl ester composition of lower alkyl linoleate esters. The catalyst can be delivered as a solid or as a solution in the conjugate alcohol of the alkoxide.

The isomerization step can be performed at temperatures low enough to suppress formation of undesirable CLA isomers but sufficient to cause rearrangement of the double bonds. Such temperatures can be at or below 140 °C, alternatively between 90-130 °C, alternatively between 110-120 °C, and alternatively at about 120 °C. The catalyst can be added to the alkyl ester composition at 140 °C or below.

In accordance with a further embodiment of the present technology, no solvent is added for the isomerization step. The catalyst for the isomerization step may be added in a solvent, but the alkyl ester of the linoleic acid-containing material is not dissolved in a solvent. Relative to the ester quantity, the catalyst solvent is present in a minimal and negligible amount at any given time since the catalyst solvent is distilled from the reactor soon after it is added. By avoiding the use of solvent in the isomerization step, the potential formation of unwanted CLA-alcohol esters is eliminated.

However, a person of ordinary skill in the art will understand that the process of the invention can optionally, be carried out in the presence of solvents which do not interfere with the overall conjugation reaction. Examples of such optional solvents, which can be used in an amount of 10 to 50 percent based on the weight of the alkyl ester composition, are methyl, ethyl, isopropyl, butyl, amyl alcohol, pentane, hexane, heptane, heptylene-(1), octylene-1, benzene, toluene, or a combination thereof.

In accordance with another embodiment of the present invention, the transesterification and isomerization steps can be performed in one reaction vessel either concurrently or sequentially, without an intervening distillation step to refine the alkyl ester composition resulting from the transesterification step. The transesterification and isomerization reactions can occur concurrently in a continuous reaction system using a dual reaction zone apparatus, and the transesterification side products can be substantially removed via a centrifuge (for example, a Lavin centrifuge from AML Industries, Inc., (Hatboro, PA)), an in-line phasing unit, or a separate phasing vessel. An in-line phasing system allows the separation of immiscible parts of a stream by providing a region for continuous decanting set up such that additional motive force is not necessary to move the desired process phase out of the phasing system. Also, when a continuous reaction system using a dual reaction zone apparatus is employed, the transesterification step can occur in the first reaction zone, while the isomerization step can be completed in the second reaction zone. The individual reaction zones can be envisioned as a heated length of piping equipped with in-line mixing sections or as a stirred reaction vessel.

Additionally, the refined ester stream enriched in desirable conjugated linoleate esters can be further processed, for example, in accordance with the saponification and neutralization steps described in U.S. Patent Application No. 10/434,011.

In the saponification step, the refined CLA ester stream enriched in CLA-containing fatty acid ester can react with an inorganic hydroxide or an alkyl ammonium hydroxide to produce a CLA-containing fatty acid salt. The saponification step can be performed between ambient temperature and 100 °C, alternatively between 45 °C to 100 °C. The cation of the inorganic hydroxide can be sodium (Na), potassium (K) or calcium (Ca), and the cation of the alkyl ammonium hydroxide can be a symmetrical lower tetraalkyl (1-4 carbons) (tetramethyl, tetraethyl, tetrapropyl and tetrabutyl), benzyl trialkyl (1-4 carbons), dibenzyl dialkyl (1-4 carbons) or long chain alkyl (12-18 carbons) trialkyl (1-4 carbons) ammonium group. The hydroxide/ester ratio can be within the range of 1.05-2.5, and preferably within the range of 1.05-1.5.

The saponification step can be performed in an aqueous or nonaqueous aliphatic mono-alcohol, or mixed aqueous/alkyl mono-alcohol system. Examples of such solvents include, but are not limited to, water, methanol, ethanol, isopropanol, butanol and combinations thereof.

In the optional neutralization step, a concentrated acid is added to the CLA-containing fatty acid salt solution to liberate the CLAs. Examples of suitable acids for the neutralization are sulfuric, phosphoric, hydrochloric, citric and oxalic acids.

The CLA-containing fatty acid esters or other CLA products or derivatives resulting from the presently described technology, are enriched in desirable cis-9, trans-11 (c9,t11) and trans-10,cis-12 (t10,c12)-CLA isomers, but contain very small amounts of undesirable isomers, unconjugated linoleic acid components, and other side products or impurities. Such superior CLA products or derivatives have wide nutritional, therapeutic, pharmacological or other uses as those are currently known or will be known in the art.

The presently described technology and its advantages will be better understood by reference to the following examples. These examples are provided to describe specific embodiments of the present technology and to demonstrate how it works. By providing those specific examples, the inventors do not limit the scope of the present technology. It will be understood by those skilled in the art that the full scope of the presently described technology encompasses the subject matter defined by the claims concluding this specification.

### Examples

### comparative examples

### Example 1: Purification of Conjugated Linoleic Acid Methyl Esters via Distillation

A conjugated linoleic acid methyl ester (CLME) stream was distilled via two passes in a thin film evaporator, which is a low residence time distillation apparatus. Distillation conditions were: oil temperature range of 120-125 °C; reduced system pressure of 6.666-13.32 Pa (0.05-0.1 mm Hg.) The initial CLME stream composition was methyl palmitate (C16:0): 6.20%; methyl stearate (C18:0): 2.37%; methyl oleate (C18:1): 12.65%; methyl linoleate (unconjugated C18:2): 2.42%; CLME (conjugated C18:2): 75.00%. After the two pass distillation run, the CLME stream composition was methyl palmitate: 2.69%; methyl stearate: 2.34%; methyl oleate: 11.37%; methyl linoleate: 2.14; CLME: 80.34%. The fatty acid distribution was determined by GC, and no thermal rearrangement products were detected after the distillation operation.

Comparing the CLME content of the ester stream after distillation to that before distillation, the data represents an increase of 7.1% in the CLME content. The data also shows that the contents of other components were all decreased.

### Example 2: Purification of Safflower Oil Methyl Esters via Distillation

In this example, a composition containing unconjugated linoleic acid methyl esters was distilled, and the result was compared with that of Example 1 in which a stream containing conjugated linoleic acid methyl esters was distilled using the same distillation apparatus.

A safflower oil methyl ester (SOME) stream was distilled via two passes in a thin film evaporator, which is a low residence time distillation apparatus. Distillation conditions were: oil temperature range of 115-125 °C ; reduced system pressure of 6.66-13.32 Pa (0.05-0.1 mm Hg). The initial SOME stream composition was methyl palmitate: 6.22%; methyl stearate: 2.34%; methyl oleate: 12.50%; linoleic acid methyl ester (methyl linoleate): 76.38%. After the two pass distillation run, the SOME stream composition was methyl palmitate: 3.78%; methyl stearate: 2.61%; methyl oleate: 13.01%; methyl linoleate: 78.12%. The fatty acid distribution was determined by GC.

The data represents an increase of only 2.2% in the methyl linoleate content of the ester stream after distillation. A comparison of Examples 1 and 2 reveals that the increase in the desired esters components obtained from the CLME distillation was over 300% greater than that obtained by the SOME distillation (7.1% vs. 2.2%).

### Example 3: Thermal Stability Study of SOME at 200 °C

SOME was held at 200 °C under nitrogen for 50 hours. The solution was sampled intermittently and analyzed by GC. After 50 hours, the methyl linoleate content (c9, c12 isomer) decreased from 78.0% to 77.0%. Other unconjugated linoleate isomers (c9, t12; t9, c12) grew from 0.49% and 0.48% to 0.82% and 0.76%, respectively. No increase in conjugated linoleate esters was observed. This example illustrates that unconjugated SOME is substantially stable thermally.

### Example 4: Thermal Stability Study of CLME at 195 °C

CLME was held at 195 °C under nitrogen. Samples were taken and analyzed by GC. After 77 minutes at 195 °C, the amount of the c11,t13 isomer of CLME grew from nondetectable to 2.3%. No increase in unconjugated linoleate esters was observed. Comparison of the results of Examples 3 and 4 highlights the differences between CLA esters and SOME. These esters have different thermal stabilities and undergo significantly different thermal rearrangement pathways.

### Example 5: Conversion of Safflower Oil to CLME

Safflower oil (421.38 g, 0.487 mol) was combined with methanol (93.19 g, 2.909 mol). A 25% solution of sodium methoxide (8.38 g, 0.5 wt % active) was added at 65 °C. After 1 hour, the lower phase was removed. The organic phase was washed, dried under vacuum and then heated to 120 °C. A 25% solution of potassium methoxide (33.4 g, 2% wt) in methanol was added to the ester solution at 120 °C. After completion of the conjugation reaction, the catalyst was neutralized with dilute citric acid solution. The CLME product was washed once with water and dried under vacuum. This example shows one way to convert a linoleic acid-rich oil to a composition rich in conjugated linoleic acid esters via a transesterification step followed by an isomerization step.

### Example 6: Purification of CLME via Distillation through A Rectification Column

A CLME stream was distilled in a unit consisting of a thin film evaporator connected to a rectification column (25.4 cm of cm of packing) (10 inches of packing). Distillation conditions were: still heater temperature range 240-270 °C; system pressure 46.7-66.6 Pa (0.35-0.5 mm Hg) (top of the column). The initial CLME stream composition was methyl palmitate: 3.96%; methyl stearate: 2.62%; methyl oleate: 14.57%; methyl linoleate (unconjugated C18:2): 1.00%; CLME (conjugated C18:2): 74.84%. At the end of the distillation run, the bottoms stream composition was methyl palmitate: 0.46%; methyl stearate: 2.36%; methyl oleate: 10.56%; methyl linoleate: 0.79%; CLME: 83.03%. The fatty acid distribution was determined by GC. No thermal rearrangement products were detected after the distillation operation.

This data represents an increase of 10.9% in the CLME content of the ester stream after distillation. A comparison of Examples 2 and 6 reveals that the increase in the desired esters components obtained from the CLME distillation operated in the mode of Example 6 was nearly 500% greater than that obtained by the SOME distillation (10.9% vs. 2.2%).

The results of Examples 1 and 6 illustrate that although CLA esters may have a reduced thermal stability compared to SOME, CLA esters still can be safely distilled under controlled conditions without undergoing significant thermal rearrangements.

The invention is now described in such full, clear, concise and exact terms as to enable any person skilled in the art to which it pertains, to practice the same.

## Claims

1. A process to refine a conjugated linoleic acid-containing material comprising:
distilling a first ester stream containing esters of conjugated linoleic acids using a distillation apparatus which is a thin-film or wiped-film evaporator and contains a fractionating column; and
producing a second ester stream enriched in the esters of conjugated linoleic acids.

2. The process of claim 1, wherein the distilling step uses a single or multi-pass distillation operation.

3. The process of claim 1, wherein the distillation apparatus is operated at a reduced pressure of greater than 0 and lower than 101.325 kPa [760 mmHg]

4. The process of claim 1, further comprising the step of at least partially removing side products generated during the formation of the first ester stream.

5. The process of claim 1, further comprising the step of at least partially removing unconjugated linoleic acid components in the first ester stream.

6. A process to produce a refined conjugated linoleic acid-containing material, comprising:
transesterification of a linoleic acid-containing oil to generate a composition containing linoleic acid esters;
isomerization of the composition containing linoleic acid esters to form a first stream containing conjugated linoleic acid esters; and
distillation of the first stream using a distillation apparatus which is a thin-film or wiped-film evaporator and contains a fractionating column to produce a second stream enriched in conjugated linoleic acid esters.

7. The process of claim 6, wherein the distillation apparatus is capable of being operated at a reduced pressure, of lower than 101,325 kPa[760 mmHg].

8. The process of claim 6, wherein the step of isomerization is catalyzed by a catalyst base in a nonaqueous system.

9. The process of claim 8, wherein the catalyst base is an alkali or alkaline earth alkoxide salt of a C₁-C₄ alkyl group alcohol.

10. The process of claim 9, wherein the cation of the alkoxide salt is a sodium, a potassium or a calcium cation.

11. The process of claim 8, wherein the catalyst base is a solid or a solution in a conjugate alcohol of the alkoxide.

12. The process of claim 6, wherein the step of isomerization is performed between 90-140 °C.

13. The process of claim 6, wherein the step of isomerization is performed between 110-120 °C.

14. The process of claim 6, wherein the linoleic acid-containing oil is selected from the group consisting of safflower oil, corn oil, sunflower oil, soybean oil, grape seed oil, cottonseed oil, sesame oil, derivatives thereof, and combinations thereof.

15. The process of claim 6, wherein the transesterification and isomerization steps are performed in one reaction vessel concurrently or sequentially without an intervening distillation step.

16. The process of claim 6, wherein the transesterification and isomerization steps occur concurrently in a continuous reaction system using a dual reaction zone apparatus.

17. The process of claim 16, further comprising the step of at least partially removing side products from the transesterification step.

18. The process of claim 16, wherein the transesterification step is completed in a first reaction zone and the isomerization step is completed in a second reaction zone.

## Patentansprüche

1. Verfahren zum Raffinieren eines konjugierte Linolsäure-enthaltenden Materials, umfassend:
Destillieren eines ersten Ester-Stroms, der Ester von konjugierten Linolsäuren enthält, unter Verwendung einer Destillationsapparatur, die ein Dünnschicht- oder Wischfilm-Verdampfer ist und eine Fraktionierkolonne enthält; und
Herstellen eines zweiten Ester-Stroms, der mit den Estern von konjugierten Linolsäuren angereichert ist.

2. Verfahren nach Anspruch 1, wobei der Destillationsschritt einen einfach oder mehrfach ablaufenden Destillationsvorgang verwendet.

3. Verfahren nach Anspruch 1, wobei die Destillationsapparatur bei einem verminderten Druck von mehr als 0 und weniger als 101,325 kPa [760 mmHg] betrieben wird.

4. Verfahren nach Anspruch 1, das ferner den Schritt eines zumindest teilweisen Entfernens von Nebenprodukten, die während der Bildung des ersten Ester-Stroms erzeugt werden, umfasst.

5. Verfahren nach Anspruch 1, das ferner den Schritt eines zumindest teilweisen Entfernens von unkonjugierte Linolsäure-Bestandteilen in dem ersten Ester-Strom umfasst.

6. Verfahren zum Herstellen eines raffinierten, konjugierte Linolsäure-enthaltenden Materials, umfassend:
Umesterung eines Linolsäure-enthaltenden Öls, um eine Zusammensetzung, die Linolsäure-Ester enthält, zu erzeugen;
Isomerisierung der Zusammensetzung, die Linolsäure-Ester enthält, um einen ersten Strom, der konjugierte Linolsäure-Ester enthält, zu erzeugen; und
Destillation des ersten Stroms unter Verwendung einer Destillationsapparatur, die ein Dünnschicht- oder Wischfilm-Verdampfer ist und eine Fraktionierkolonne enthält, um einen zweiten Strom herzustellen, der mit konjugierte Linolsäure-Estern angereichert ist.

7. Verfahren nach Anspruch 6, wobei die Destillationsapparatur bei einem verminderten Druck von weniger als 101,325 kPa [760 mmHg] betriebsfähig ist.

8. Verfahren nach Anspruch 6, wobei der Schritt einer Isomerisierung von einer Katalysator-Base in einem nicht-wässrigen System katalysiert wird.

9. Verfahren nach Anspruch 8, wobei die Katalysator-Base ein Alkali- oder Erdalkali-Alkoxidsalz eines Alkohols mit einer C₁-C₄-Alkylgruppe ist.

10. Verfahren nach Anspruch 9, wobei das Kation des Alkoxidsalzes ein Natrium-, ein Kalium- oder ein Kalzium-Kation ist.

11. Verfahren nach Anspruch 8, wobei die Katalysator-Base ein Feststoff oder eine Lösung in einem konjugierten Alkohol des Alkoxids ist.

12. Verfahren nach Anspruch 6, wobei der Schritt einer Isomerisierung zwischen 90-140 °C durchgeführt wird.

13. Verfahren nach Anspruch 6, wobei der Schritt einer Isomerisierung zwischen 110-120 °C durchgeführt wird.

14. Verfahren nach Anspruch 6, wobei das Linolsäure-enthaltende Öl ausgewählt ist aus der Gruppe, bestehend aus Färberdistelöl, Maisöl, Sonnenblumenöl, Sojaöl, Traubenkernöl, Baumwollkernöl, Sesamöl, Derivaten davon und Kombinationen davon.

15. Verfahren nach Anspruch 6, wobei die Umesterungs- und Isomerisierungsschritte in einem Reaktionsgefäß gleichzeitig oder der Reihe nach ohne einen dazwischenliegenden Destillationsschritt durchgeführt werden.

16. Verfahren nach Anspruch 6, wobei die Umesterungs- und Isomerisierungsschritte gleichzeitig in einem kontinuierlichen Reaktionssystem unter Verwendung einer Apparatur mit einer dualen Reaktionszone stattfinden.

17. Verfahren nach Anspruch 16, das ferner den Schritt eines zumindest teilweisen Entfernens von Nebenprodukten aus dem Umesterungsschritt umfasst.

18. Verfahren nach Anspruch 16, wobei der Umesterungsschritt in einer ersten Reaktionszone vollendet wird und der Isomerisierungsschritt in einer zweiten Reaktionszone vollendet wird.

## Revendications

1. Procédé pour le raffinage d'une matière contenant de l'acide linoléique conjugué comprenant:
la distillation d'un premier courant d'ester contenant des esters d'acides linoléiques conjugués au moyen d'un appareil de distillation qui est un évaporateur à film mince ou à film essuyé et contient une colonne de fractionnement; et
la production d'un deuxième courant d'esters enrichi en les esters d'acides linoléiques conjugués.

2. Procédé selon la revendication 1, dans lequel l'étape de distillation utilise une opération de distillation avec passage unique ou passages multiples.

3. Procédé selon la revendication 1, dans lequel l'appareil de distillation est mis en fonctionnement sous une pression réduite de plus de 0 et moins de 101,325 KPa [760 mm de Hg].

4. Procédé selon la revendication 1, comprenant en outre l'étape d'élimination au moins partielle des produits secondaires générés pendant la formation du premier courant d'esters.

5. Procédé selon la revendication 1, comprenant en outre l'étape d'élimination au moins partielle des composants d'acides linoléiques non conjugués dans le premier courant d'esters.

6. Procédé pour la production d'une matière raffinée contenant des acides linoléiques conjugués, comprenant:
la transestérification d'une huile contenant des acides linoléiques pour générer une composition contenant des esters d'acides linoléiques;
l'isomérisation de la composition contenant des esters d'acides linoléiques pour former un premier courant contenant des esters d'acides linoléiques conjugués; et
la distillation du premier courant en utilisant un appareil de distillation qui est un évaporateur à film mince ou à film essuyé et contient une colonne de fractionnement pour produire un deuxième courant enrichi en esters d'acides linoléiques conjugués.

7. Procédé selon la revendication 6, dans lequel l'appareil de distillation est apte à être mis en oeuvre à une pression réduite inférieure à 101,325 KPa [760 mm de Hg].

8. Procédé selon la revendication 6, dans lequel l'étape d'isomérisation est catalysée par une base catalytique dans un système non aqueux.

9. Procédé selon la revendication 8, dans lequel la base catalytique est un alcali ou un sel alcoxyde de métal alcalin ou alcalino-terreux d'un alcool avec groupe alkyle en C₁-C₄.

10. Procédé selon la revendication 9, dans lequel le cation du sel alcoxyde est un cation sodium, potassium ou calcium.

11. Procédé selon la revendication 8, dans lequel la base catalytique est un solide ou une solution dans un alcool conjugué de l'alcoxyde.

12. Procédé selon la revendication 6, dans lequel l'étape d'isomérisation est mise en oeuvre entre 90-140°C.

13. Procédé selon la revendication 6, dans lequel l'étape d'isomérisation est mise en oeuvre entre 110-120°C.

14. Procédé selon la revendication 6, dans lequel l'huile contenant des acides linoléiques est choisie dans le groupe consistant en huile de carthame, huile de maïs, huile de tournesol, huile de soja, huile de pépins de raisin, huile de graine de coton, huile de sésame, leur dérivés, et leurs combinaisons.

15. Procédé selon la revendication 6, dans lequel les étapes de transestérification et d'isomérisation sont mises en oeuvre dans un réacteur concurremment ou séquentiellement sans faire intervenir une étape de distillation.

16. Procédé selon la revendication 6, dans lequel les étapes de transestérification et d'isomérisation interviennent concurremment dans un système de réaction en continu utilisant un appareil à zone de réaction duale.

17. Procédé selon la revendication 16, comprenant en outre l'étape d'élimination au moins partielle de sous-produits de l'étape de transestérification.

18. Procédé selon la revendication 16, dans lequel l'étape de transestérification est accomplie dans une première zone de réaction et l'étape de d'isomérisation est accomplie dans une deuxième zone de réaction.
